# EUROPEAN PATENT SPECIFICATION

(11) **EP 1 644 072 B1**
(45) Date of publication and mention of the grant of the patent: **28.09.2011**
(21) Application number: 04737899.7
(22) Date of filing: 28.06.2004
(51) Int. Cl.: A61M 39/14

(54) **Coupling device for medical lines**
Kupplungsvorrichtung für medizinische Leitungen
Raccord pour tuyaux medicaux

(30) Priority: 14.07.2003 US 619325; 28.10.2003 US 514615 P
(43) Date of publication of application: 12.04.2006
(73) Proprietor: B & D Research and Development Inc., Rimbey, Alberta T0C 2J0 (CA)
(72) Inventor: COAMBS, David, John, Rimbey, Alberta T0C 2J0 (CA); WRIGHT, Bridget, Adele, Rimbey, Alberta T0C 2J0 (CA)
(74) Representative: Neilson, Martin Mark
(86) International application number: PCT/CA2004/000960
(87) International publication number: WO 2005/004974

(56) References cited:
- WO-A-02/087682
- US-A- 4 334 551
- US-A- 4 610 469
- US-A- 5 492 147
- US-A- 5 492 147
- US-A- 5 957 898
- US-A- 6 146 374
- US-A- 6 146 374

## Description

### FIELD OF THE INVENTION

This invention relates to medical lines and, in particular, to a coupling device for connecting two medical lines.

### BACKGROUND OF THE INVENTION

Medical lines are commonly used to deliver liquids or gases to or from a patient under medical care. Medical fluid lines are used regularly in conjunction with a catheter for the intravenous (IV) delivery of fluids, often including medication. They may also be used for fluid drainage, such as in the case of a urinary catheter. Oxygen lines are often used to deliver oxygen to patients to assist in breathing.

One of the dangers with medical fluid lines attached to a patient through a catheter is that they can become snagged or entangled on external objects or persons. This can cause the patient pain and physical damage if a medical fluid line is snagged during a fall or some other rapid movement, since the catheter may be torn from the insertion site on the patient. Alternatively, the fluid line may be torn from the IV bag or other equipment to which it is attached. In either case, spillage of body fluids or medicaments or the contamination of the fluid lines are significant risks.

Another danger arises in the use of IV lines with newborns and infants. In some cases, infants can be strangled by IV lines if the child becomes entangled in the IV line. This danger also arises in the case of medical lines for the delivery of gases, such as oxygen lines.

There are existing two-part connectors for coupling medical fluid lines, however these connectors fail to adequately address the problems noted above. Existing two-part connectors are designed to lock together until manually detached by a nurse. For example, US Patent Nos. 5,549,577 and 5,122,123 and US Patent Publication Nos. 20030032940 and 200200123724, each contemplate a threaded attachment or a bayonet-style attachment which are intended to lock the connector into place.

US Patent Nos. 4,533,349 and 5,637,088 describe connectors or fluid lines that can become detached as a result of a longitudinal pulling force, but detachment results in spillage of fluids and risk of contamination.
US Patent no. 4,334,551 describes mating terminal connectors that provide sterile connections without spillage of fluids. However, the connection between the two connectors is a two-step process involving first telescopically receiving one end of a connector in the corresponding open end of the second connector and then rotating and advancing a plunger seated in the second connector to establish fluid communication therebetween.

Accordingly, a need exists for a coupling device for medical lines that, in part, addresses the shortcomings described above.

### SUMMARY OF THE INVENTION

The present invention provides a coupling device with a safety breakaway feature. In particular, the present invention provides a coupling device having two parts that couple together to connect two medical lines and that automatically decouple when subjected to a predetermined separating force.

In one embodiment, the parts are connected together by way of a detachable snap-fit mechanism that separates when subjected to a predetermined longitudinal force. In a further embodiment, the parts each include a sealing mechanism for sealing the medical lines when the parts are coupled.

According to one aspect of the present invention there is provided a coupling device for coupling a patient-side medical line to an equipment-side medical line, said coupling device comprising: a first part adapted to be coupled to a first medical line, said first part having a first passage therethrough to provide fluid communication with said first medical line, said first part including a first seal having a sealed position and an unsealed position, wherein said first seal seals said first medical line when in said sealed position; and a second part adapted to be coupled to a second medical line, said second part having a second passage therethrough to provide fluid communication with said second medical line, said second part including a second seal having a sealed position and an unsealed position, wherein said second seal seals said second medical line when in said sealed position,wherein said parts include a connector detachably connecting said first part to said second part in a longitudinal direction, said first part receiving said second part, wherein said connector detaches said first and second parts in response to a predetermined force in said longitudinal direction, and wherein each of said seals moves from said sealed position to said unsealed position when said first part is detachably connected to said second part, said first part including a protrusion for pushing said second sealing means into an unsealed position when said first and second parts are connected.

In one embodiment, a coupling device includes a first body for coupling a patient-side medical line to an equipment-side medical line. The coupling device includes a first body having a first passage therethrough for coupling to a first medical line, a second body having a spare second passage therethrough for coupling to a second medical line, and a connection means for detachably connecting the first body to the second body and providing fluid communication between the first and second medical lines, the connection means disconnecting the first body from the second body in response to a separating force. The first body includes a first sealing means for sealing the first medical line and the second body includes a second sealing means for sealing the second medical line when the first and second bodies are disconnected, and for unsealing the first and second medical lines when the first and second bodies are connected.

In a further embodiment, there is provided a first adapter for a coupling to a second adapter of a coupling device for coupling a patient-side medical line to an equipment-side medical line, the second adapter having a second body adapted to be coupled to a second medical line. The first adapter includes a first body adapted to be coupled to a first medical line, the first body having a first passage therethrough to provide fluid communication with the first medical line, the first body including a first seal having a sealed position and an unsealed position, wherein the first seal seals the first medical line when in the sealed position, the first body including a connector for detachably connecting the first body to the second body in a longitudinal direction, the connector detaching the first and second bodies in response to a predetermined force in the longitudinal direction, wherein each of the seals moves from the sealed position to the unsealed position when the first body is detachably connected to the second body.

In one embodiment, the connector connecting the first part or body to the second part or body includes a snap-fit connector comprising a ridge and a corresponding groove. In another embodiment, the snap-fit connector comprises a first ridge and a first corresponding groove and a second ridge and a second corresponding groove, wherein the second ridge is selectably engagable with the second corresponding groove so as to allow a user to select between two or more predetermined detachment forces.

In yet other embodiments, the present invention may include a backflow prevention mechanism or valve, and may include an external med-port.

Other aspects and features of the present invention will become apparent to those ordinarily skilled in the art upon review of the following description of specific embodiments of the invention in conjunction with the accompanying figures.

### BRIEF DESCRIPTION OF THE DRAWINGS

Reference will now be made, by way of example, to the accompanying drawings which show an embodiment of the present invention, and in which:

Figure 1 shows a perspective view of a coupling device according to the present invention in a disconnected state;

Figure 2 shows a cross-sectional view of the coupling device shown in Figure 1 taken along the line 2-2, with the device in a disconnected state;

Figure 3 shows a cross-sectional view of the coupling device shown in Figure 1 taken along the line 2-2, with the device in a connected state;

Figure 4 shows a close-up of the cross-sectional view of one of the diaphragms shown in Figure 2;

Figure 5 shows a perspective view of another embodiment of a coupling device according to the present invention, in a disconnected state;

Figure 6 shows a cross-sectional view of a third embodiment of a coupling device according to the present invention;

Figure 7 shows a cross-sectional view of a fourth embodiment of a coupling device according to the present invention;

Figure 8 shows a cross-sectional view of a fifth embodiment of a coupling device according to the present invention; and

Figure 9 shows a cross-sectional view of a sixth embodiment of a coupling device according to the present invention.

Similar numerals are used in different figures to denote similar components.

### DESCRIPTION OF SPECIFIC EMBODIMENTS

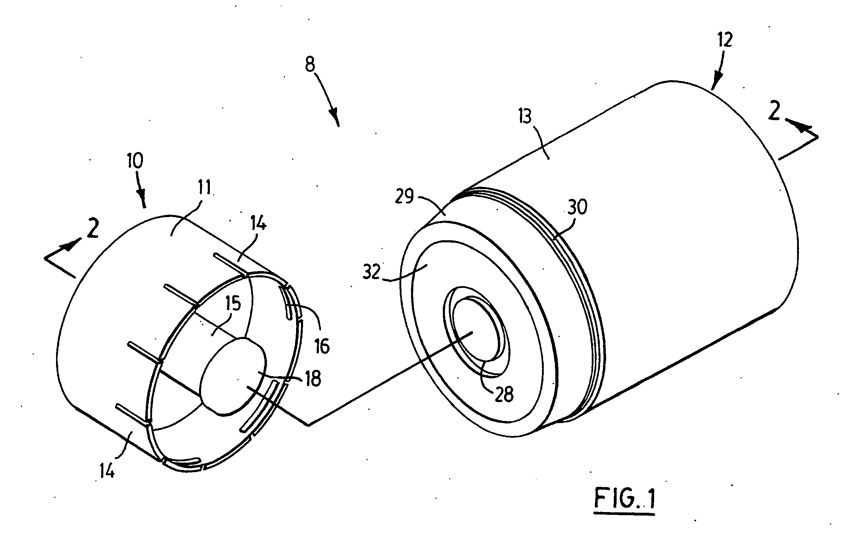

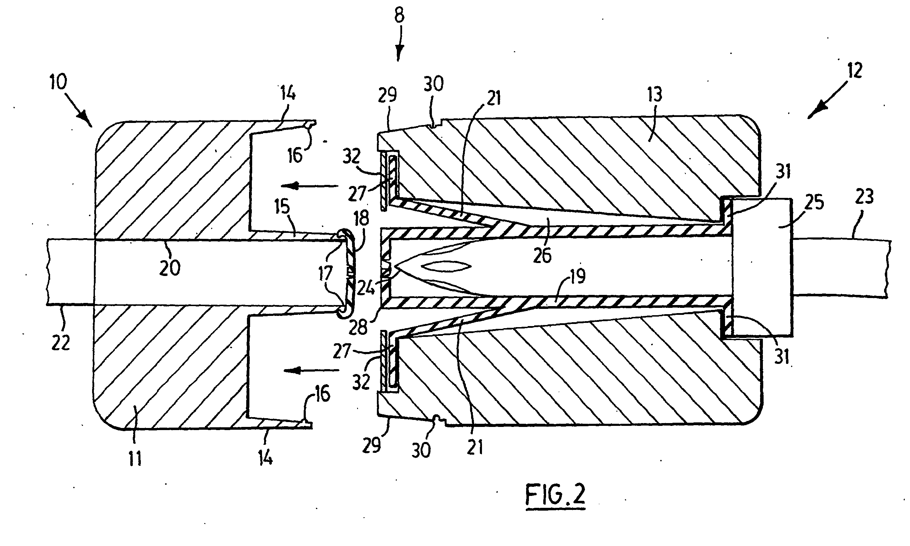

Reference is first made to Figures 1 and 2, which show an embodiment of a coupling device 8, according to the present invention, in a disconnected state. The coupling device 8 includes a first, or female, part 10 and a second, or male, part 12. The parts 10 and 12 each have a forward or proximal end where the two parts 10 and 12 are intended to meet and a back or distal end remote from the forward or proximal end. The parts 10 and 12 are adapted to be coupled together at their proximal ends such that they will disengage when subjected to a predetermined longitudinal force, as is further described herein.

The female part 10 has a cylindrical body 11 having an axial passage 20 extending through the body 11. At the distal end of the body 11, the passage 20 is in fluid communication with a fluid line 22. The fluid line 22 may be coupled to the passage 20 through any number of mechanisms for securing the female part 10 to the fluid line 22, including a barbed connector, crimping, a threaded coupling, a bayonet-style coupling, or a fused connection. The part 10 may, in one embodiment, include a bayonet termination for insertion directly into an IV solution bag to provide fluid communication with the passage 22. In one embodiment, the female part 10 and/or the male part 12 are not secured directly to the fluid lines. In this embodiment, the parts 10, 12 terminate with a connectors which is adapted to be coupled to a corresponding connector on the fluid lines, such as a threaded connector or other known connectors.

Extending forward from the proximal end of the body 11 is an axial tube 15 in fluid communication with the passage 20. The axial tube 15 may be integrally formed with the cylindrical body 11 and terminates in an outer end 17. The outer end 17 of the tube is sealed with a first diaphragm 18. Accordingly, the first diaphragm 18 also seals the passage 20 and the fluid line 22, thereby preventing fluid flow into or out of the fluid line 22. In one embodiment, the diaphragm 18 includes a pre-cut central slit, whereby the diaphragm 18 spreads open at the pre-cut central slit when subjected to sufficient pressure.

The female part 10 also includes a plurality of forward extending arms 14 arranged at the periphery of the cylindrical body 11. The arms 14 may be integrally formed with the cylindrical body 11. The outer ends of some of the arms 14 include a ridge 16 formed on the inner surface of the arms 14. As can be seen in Figure 1, the ridge 16 may extend circumferentially along the inner surface of one or more arms 14. The arms 14 are resiliently flexible such that when bent radially outwards they will exert an inward radial bias.

The male part 12 has a cylindrical body 13 having an axial chamber 26 extending longitudinally therethrough. A needle 24 (or a tube, a cannula, or other fluid line connector) is coupled to a fluid line 23 and inserted into the axial chamber 26. In one embodiment, the needle 24 and fluid line 23 are secured to the cylindrical body 13 by a threaded coupler 25 having an external thread on its outer surface that cooperates with a corresponding thread formed upon the inner surface of the axial chamber 26 to secure the coupler 25 to the cylindrical body 13. Other mechanisms for coupling the fluid line 23 and needle 24 to the body 13 will be understood by those of ordinary skill in the art, and may include friction fit, adhesives, fusing, etc.

The male part 12 further includes a tubular sheath 19 disposed within the axial chamber 26. The sheath 19 envelopes the needle 24 and includes a base having an outwardly extending flange 31. The outwardly extending flange 31 is pinched between the cylindrical body 13 and the threaded coupler 25 such that the sheath 19 is in sealed fluid communication with the needle 24.

The tip of the sheath 19 terminates in a second diaphragm 28 that seals the tip of the tubular sheath 19. The diaphragm 28 may be formed integrally with the sheath 19. In one embodiment, the diaphragm 28 includes a pre-cut central slit, whereby the diaphragm 28 spreads open at the pre-cut central slit when subjected to sufficient pressure.

The sheath 19 also includes a skirt 21 encircling the tubular portion of the sheath 19 and extending forwardly and outwardly within the axial chamber 26. The outer end of the skirt 21 includes an outwardly extending flange 27. pressed against the front surface of the cylindrical body 13 and held in place with a collar 32. The collar 32 may be push fit into a corresponding annular depression within the front face of the cylindrical body 13. The collar 32 holds the outer end of the skirt 21 in place relative to the cylindrical body 13.

The front end of the cylindrical body 13 includes an inwardly tapered outer surface 29 and a circumferential groove 30.

When the parts 10 and 12 are not coupled together, the diaphragms 18 and 28 are sealed, preventing any fluid from flowing into or out of the fluid lines 22 and 23 through the passage 20 or the axial chamber 26.

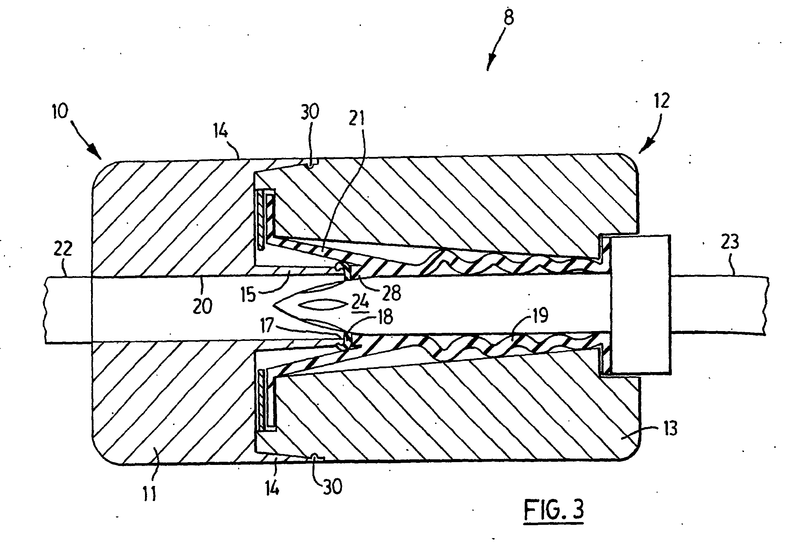

Reference is now made to Figure 3, which shows the coupling device 8 of Figures 1 and 2 in a connected state.

When the forward or proximal ends of the female and male parts 10 and 12 are brought together, the inwardly tapered outer surface 29 of the male part 12 fits within the arms 14 of the female part 10. As the ridges 16 on the arms 14 are brought into contact with the inwardly tapered outer surface 29, they are pushed outwards, flexing the arms 14 radially, until the ridges 16 snap into the groove 30. Accordingly, the two parts 10 and 12 are adapted to snap-fit together.

The groove 30 and the ridges 16 are rounded, such that they will disengage when subjected to a sufficient longitudinal force. This disengagement force may be set at different levels for different uses of the coupling device 8 (e.g. adults versus infants; urinary catheters versus IV lines; etc.). Examples of possible force levels include 1, 5, and 10 lbs. Such a force may arise if one of the fluid lines 22 or 23 is caught on an external object or tugged, thereby transferring force into the two parts 10 and 12. If the force is strong enough, it will cause the rounded edge of the ridges 16 to bear against the rounded edge of the groove 30, causing the ridges 16 to rise out of the groove 30 against the inward bias of the resiliently flexible arms 14. The extent of the force required to separate the two parts 10 and 12 is configurable by altering the relative shapes of the ridges 16 and the grooves 30 and altering the flexibility of the arms 14.

It will also be seen from Figure 3 that when the two parts 10 and 12 are connected, the outer end 17 of the axial tube 15 bears against the tip of the tubular sheath 18 around the periphery of the diaphragm 28. As the two parts 10 and 12 are brought together, the outer end 17 of the axial tube 15 pushes the sheath 19 and diaphragm 28 back, compacting the main body of the sheath 19 towards its base. As the tubular body of the sheath 19 is pushed back, the diaphragm 28 at the tip of the sheath 19 spreads apart at its central slit point or channel, revealing the needle 24. Both the main body of the sheath 19 and the diaphragm 28 are pushed back along the body of the needle 24 until the fluid ports of the needle 24 are exposed to the interior of the axial tube 15 of the female part 10.

The compacting of the sheath 19 by the axial tube 15 is performed against the resistance of the skirt 21 portion of the sheath 19. The outer end of the skirt 21 remains fixed to the front surface of the cylindrical body 13 of the male part 12, while the inner end of the skirt 21 where it meets the main body of the sheath 19 is pushed back into the axial chamber 26. This stretching stores a tension in the skirt 21. When the parts 10 and 12 are disengaged, the tension in the skirt 21 causes it to contract, drawing the body of the sheath 19 back up the axial chamber 26, causing the sheath 19 to reassume its sealed position enveloping the needle 24. When the sheath 19 re-envelopes the needle 24, the diaphragm 28 reseals the tip of the sheath 19, pinching the central slit shut.

It will further be seen from Figure 3 that when the two parts 10 and 12 are brought together and the outer end 17 of the axial tube 15 begins to bear upon the tip of the sheath 19 and the diaphragm 24 begins to part, the tip of the needle 24 is brought into contact with the center of the diaphragm 18 at the outer end 17 of the axial tube 15. The needle 24 exerts a pressure on the diaphragm 18, causing it to spread apart at its central slit point or channel, through which the needle 24 is inserted.

When the parts 10 and 12 are fully connected, the two diaphragms 28 and 18 are drawn apart, allowing the needle 24 to enter the axial tube 15 and fluid communication is established between the two fluid lines 22 and 23. When the two parts 10 and 12 are disconnected, the sheath 19 and the two diaphragms 28 and 18 resiliently return to their relaxed and sealed state, thereby sealing the two fluid lines 22 and 23.

Accordingly, the coupling device 8 automatically seals the two fluid lines 22 and 23 when the coupling device 8 is purposely or accidentally disconnected, thereby preventing spillage or contamination. The coupling device 8 also permits easy connection and disconnection of different fluid lines by providing easy sterilization and resealing capability. This allows for easy connection of a new IV line or new catheter bag to a patient. The coupling device 8 may also be used to provide a saline/hep lock or a med port.

Upon disconnection, in one embodiment, the coupling device 8 provides for sufficient backpressure to trigger an alarm on a pump if one is used in association with one of the medical fluid lines.

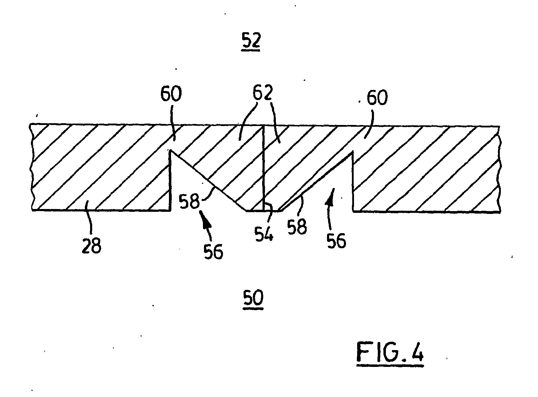

Reference is now made to Figure 4, which shows a close up of a cross-sectional view of the diaphragm 28 from the male part 12. The following description of the diaphragm 28 may also apply to the diaphragm 18 on the female part 10.

The diaphragm 28 seals an inner volume 50 from an external environment 52. It features a central slit 54 to allow the diaphragm 28 to spread apart when the two parts 10 and 12 are connected together. In many cases, the inner volume 50 contains a fluid under pressure, such that it exerts an outward pressure on the diaphragm 28.

The diaphragm 28 features one or more channels 56 formed in the inner surface of the diaphragm 28 and running parallel to the central slit 54. In one embodiment, a channel 56 is formed on each side of the central slit 54. The channels 56 each include an angled face 58 extending from the inner surface of the diaphragm 28 into the diaphragm 28 divergently from the central slit 54. The channels 56 and the central slit 54 define two hinged portions 62 of the diaphragm 28 that meet at the central slit 54. The two hinged portions 62 of the diaphragm 28 are each connected to the main part of the diaphragm 28 at a thin point 60. In another embodiment, there is a single circumferential channel 56 around a breakpoint.

Pressure from the fluid within the inner volume 50 bears against the inner surface of the diaphragm 28. This pressure also bears against the angled faces 58, urging the hinged portions 62 of the diaphragm 28 to pivot about their thin points 60, thereby compressing them together at the central slit 54 and improving the seal.

In one embodiment, the diaphragms 18 and 28, and the sheath 19 are all manufactured from silicon. Other suitable materials may include materials such as aliphatic hydrocarbon resins, aliphatic polyester resins, copolymers of olefins and vinyl acetate, olefin-acrylate copolymers, and chlorinated hydrocarbon resins, provided that they are sufficiently resiliently elastic.

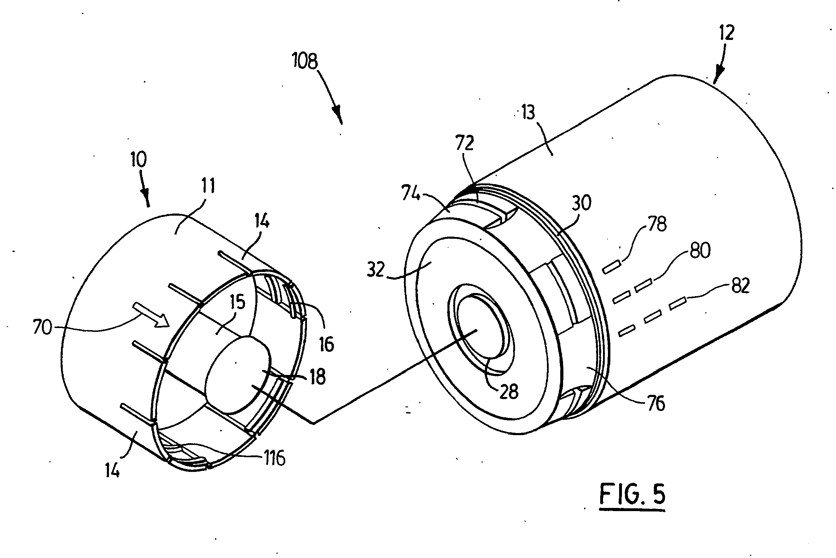

Reference is now made to Figure 5, which shows a perspective view of another embodiment of a coupling device 108, according to the present invention, in a disconnected state. The coupling device 108 includes a first, or female, part 10 and a second, or male, part 12, the parts 10 and 12 each have a forward or proximal end where the two parts 10 and 12 are intended to meet and a back or distal end remote from the forward or proximal end. The parts 10 and 12 are adapted to be coupled together at their proximal ends such that they will disengage when subjected to an adjustable predetermined longitudinal force, as is further described below.

The female part 10 includes a plurality of forward extending arms 14 arranged at the periphery of a cylindrical body 11. One such arm 14 may be marked with an alignment arrow 70. The arms 14 may be integrally formed with the cylindrical body 11. The outer ends of some of the arms 14 include a first ridge 16 and a second ridge 116 formed on the inner surface of the arms 14. As can be seen in Figure 5, the first ridge 16 and the second ridge 116 extend circumferentially along the inner surface of one or more arms 14. The arms 14 are resiliently flexible such that when bent radially outwards they will exert an inward radial bias.

The male part 12 includes a cylindrical body 13 having an inwardly tapered engagement surface 74 at its proximal end. The proximal end of the cylindrical body 13 also includes inwardly sloped clearance surfaces 76 and a circumferential groove 30. The inwardly tapered engagement surfaces 74 define a secondary circumferential groove 72. The secondary circumferential groove 72 is located closer to the proximal end of the cylindrical body 13 than the circumferential groove 30.

Cylindrical body 13 may further include first alignment marking 78, second alignment marking 80, and third alignment marking 82. The first, second, and third alignment markings 78, 80, and 82 may correspond to selectable predetermined longitudinal detachment forces, such as 1, 5, and 10 lbs., when the markings are selectably aligned with the alignment arrow 70. The alignment markings 78, 80, and 82 may correspond to different applications for the coupling device 108, such as infant, pediatric, and adult.

It will be understood that the alignment arrow 70 may be replicated at various intervals around the perimeter of female part 10, and that the first, second, and third alignment markings 78, 80, and 82 may be replicated at various intervals around the perimeter of male part 12. Further, it will be understood that there may be fewer than or more than three distinct selectable force settings and corresponding alignment markings.

When the forward or proximal ends of the female and male parts 10 and 12 are brought together such that alignment arrow 70 aligns with the first alignment marking 78, the inwardly tapered engagement surfaces 74 and inwardly sloped clearance surface 76 of the male part 12 fit within the arms 14 of the female part 10. As the first ridges 16 on the arms 14 are brought into contact with the inwardly tapered engagement surfaces 74, preferably at a point between secondary circumferential groove 72 and circumferential groove 30, they are pushed outwards, flexing the arms 14 radially, until the first ridges 16 snap into the circumferential groove 30. The extent of the minimum force required to separate the two parts 10 and 12 is configurable by altering the relative shapes of the ridges 16 and the groove 30 and altering the flexibility of the arms 14, as previously described. Accordingly, the two parts 10 and 12 are adapted to snap-fit together in a first alignment position having a minimum predetermined separation force.

The inwardly sloped clearance surfaces 76 may be configured such that the second ridges 116 do not contact or rest against inwardly sloped clearance surfaces 76 when the two parts 10 and 12 have been snap-fit together with alignment arrow 70 aligned with the first alignment marking 78.

Once connected in this manner, one of the parts 10 and 12 may be rotated relative to the other part in a clockwise direction until alignment arrow 70 aligns with the second alignment marking 80, so as to adjust and increase the predetermined separation force. On aligning the alignment arrow 70 with the second alignment marking 80, the first ridges 16 on the arms 14 remain engaged with the circumferential groove 30, ensuring that the female part 10 remains in a fixed longitudinal position relative to male part 12. As the parts 10 and 12 are rotated clockwise relative to each other, the second ridges 116 slide into alignment with the secondary circumferential grooves 72. The secondary circumferential groove 72 and the second ridges 116 may be rounded, such that they will disengage when subjected to a sufficient incremental longitudinal separation force. The incremental longitudinal separation force may be greater than the force required to disengage the first ridges 16 from the circumferential groove 30. The net predetermined separation force may be set at different levels for different uses of the coupling device 108 by adjusting the portion of the second ridges 116 that becomes engaged with the secondary circumferential grooves 72 as the female part 10 is rotated relative to the male part 12.

The net predetermined separation force may be increased by further rotating female part 10 relative to male part 12 until alignment arrow 70 aligns with the third alignment marking 82, indicating a maximum engagement between the second ridges 116 and the secondary circumferential grooves 72. A stop mechanism may be provided to prevent the further rotation of the parts 10 and 12 relative to each other, since any further rotation may be reduce the portion of the second ridges 116 engaged with the secondary circumferential grooves 72, thereby reducing the net predetermined separation force. Further, a "click" mechanism may be implemented to provide feedback to the user that alignment arrow 70 is in proper alignment with any one of or all of the first, second, or third alignment markings 78, 80, and 82.

If the net disengagement force applied to the parts 10 and 12 is strong enough to overcome the net predetermined separation force it will cause the rounded edge of the first ridge 16 to bear against the rounded edge of the circumferential groove 30, and the rounded edges of the second ridges 116 to bear against the rounded edges of the secondary circumferential grooves 72, causing the first and second ridges 16 and 116 to rise out of the grooves 30 and 71, respectively. The geometry of the inwardly tapered engagement surfaces 74 may be configured such that the inward bias of resiliently flexible arms 14 serves to push female part 10 away from male part 12, and further configured such that the ridges 16 do not catch on the secondary circumferential grooves 72 as the parts 10 and 12 are separated.

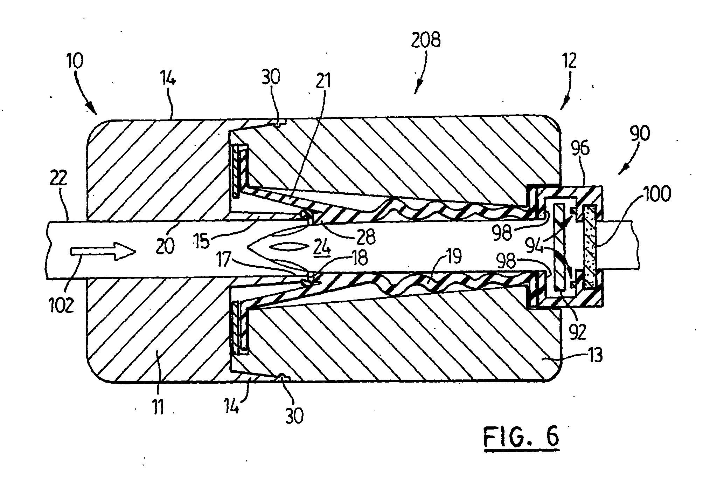

Reference is now made to Figure 6, which shows a third embodiment of a coupling device 208 according to the present invention. The coupling device 208 includes an integrated backflow prevention mechanism 90. The integrated backflow prevention mechanism 90 includes a valve body 96 defining an interior chamber with an inlet and an outlet. The backflow prevention mechanism 90 ensures fluid flow only in the direction of flow arrow 102 and substantially prevents fluid or gases from flowing in the opposite direction. A disk 92 is provided within the interior chamber of the valve body 96. The backflow prevention mechanism 90 includes disk stand-offs 94 to prevent the disk 92 from sealing an outlet passage when fluid flows in the direction of flow arrow 102. Disks stand-offs 94 may be configured to allow sufficient flow around disk 92 and between disk 92 and disk valve body 96 when disk 92 is resting upon the disk stand-offs 94. When fluid flow opposes flow arrow 102, the disk 92 bears against sealing surfaces 98 thereby sealing the inlet passage of the disk valve body 96.

Disk valve body 96 may be configured to hold a filter 100. Filter 100 may be a micro-filter type filter designed to block the flow of infectious disease agents that may be potentially contained within the fluids or gases flowing through the coupling device 208. Filter 100 is sealed against the valve body 96 to prevent any bypass flow.

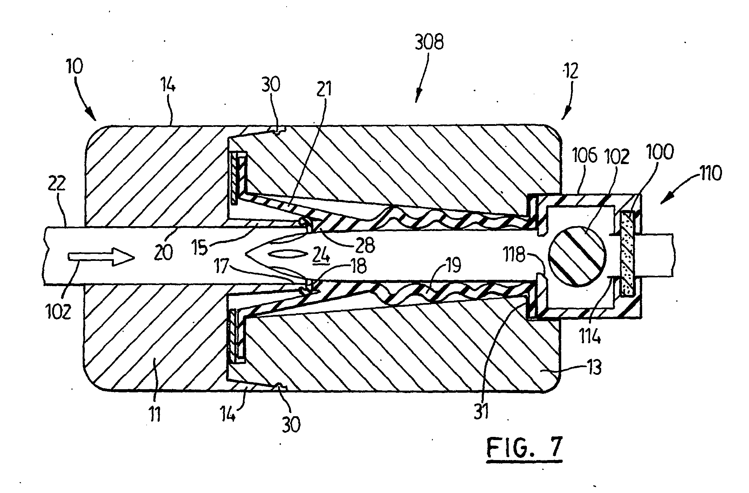

Reference is now made to Figure 7 which shows a cross-sectional view of a fourth embodiment of a coupling device 308 according to the present invention. Coupling device 308 is provided with a ball-type backflow prevention mechanism 110. The ball-type backflow prevention mechanism 110 includes a valve body 106 having an interior chamber with an inlet and outlet, and including ball stand-offs 114 and sealing surfaces 118. A ball 102 is provided within the interior chamber to ensure fluid flow only occurs in the selected direction. The ball-type backflow prevention mechanism 110 may further be provided with a filter 100, as described above.

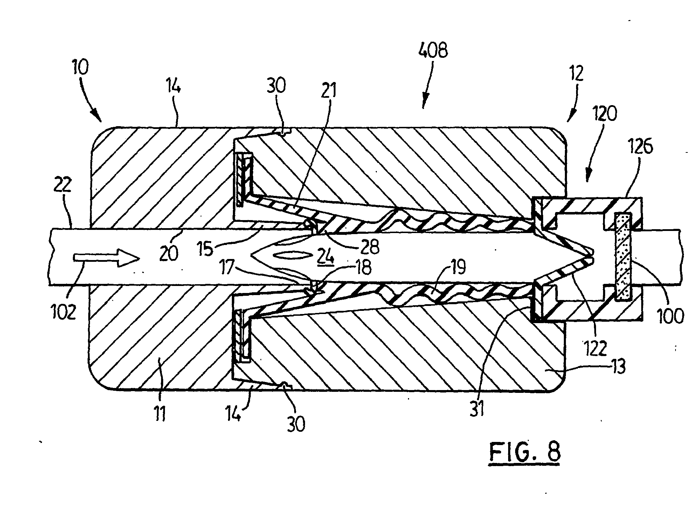

Reference is now made to Figure 8, which shows a cross-sectional view of a fifth embodiment of a coupling device 408 in accordance with the present invention. Coupling device 408 is provided with a pinch-type backflow prevention mechanism 120. The pinch-type backflow prevention. mechanism 120 includes a valve body 126 having a pinch valve 122. Pinch valve 122 may be constructed of a suitable flexible resilient material that will remain closed or "pinched" in the absence of any flow in a selected direction. Flow in a direction opposite to the selected direction causes the pinch valve 122 to close. Fluid flow against the selected direction bears against the sides of the pinch valve 122 improving the seal of the valve opening. The pinch-type of backflow prevention mechanism 120 may be configured to allow fluids or gases to flow in the direction of flow arrow 102 and to substantially prevent fluid or gases from flowing in the opposite direction.

Pinch valve 122 may be of modular construction. Pinch-type valve body 126 may be configured to seal outwardly extending flange 31 against cylindrical body 13, as previously described, seal pinch-type valve 122 against outwardly extending flange 31, and secure and seal fluid line 23 as previously described. Further, valve body 126 may be configured to hold a filter 100 in a correct and sealed position, as previously described.

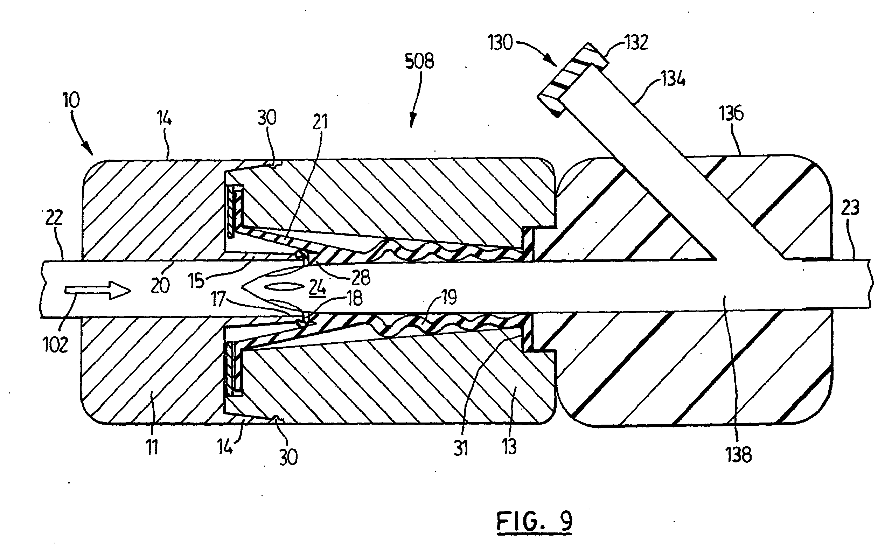

Reference is now made to Figure 9 which shows a cross-sectional view of a sixth embodiment of a coupling device 508 according to the present invention. The coupling device 508 includes an external med-port body 136 having a central passage 138 in fluid communication with the coupling device 508. The med-port body 136 further includes a tangential passage or tube 134 providing a med-port 130. External med-port 130 may be configured with a self-sealing med-port cap 132. External med-port 130 may be provided to allow for needle injections or needleless injections so as to enable fluids injected through the external med-port 130 to readily mix with fluids flowing in the direction of flow arrow 102. External med-port body 136 may be configured to seal outwardly extending flange 31 against cylindrical body 13, and secure and seal fluid line 23, as previously described.

It will be appreciated that the features and functions of external med-port 130 may be provided within cylindrical body 13 of male part 12 in an alternative embodiment. It will be further appreciated that external med-port 130 may also be configured with a backflow prevention device and/or a filter element, as previously described.

The female and male parts 10 and 12 may be manufactured by any suitable medical-grade material, including plastics having flex characteristics that are substantially unaffected by temperature variations within a reasonable operating range. The parts 10 and 12 may be produced by injection molding, or any other means known in the art.

In some embodiments the diaphragms 18 and 24 are not limited to a central slit 54 and may have multiple slits or other features for allowing the diaphragms 18 and 24 to remain sealed while the two parts 10 and 12 are disconnected and to open when the parts 10 and 12 are connected.

In some embodiments the present invention is not limited to silicon diaphragms and may include other sealing mechanisms for ensuring the two fluid lines 22 and 23 are sealed when the two parts 10 and 12 become disengaged.

In some embodiments the skirt 17 portion of the sheath 19 need not be a continuous skirt, but could be made up of two or more forward projecting arms of resiliently deformable material.

The breakaway safety feature provided by way of the snap-fit connection between the protrusions 16 and the groove 30 may be altered without affecting the function or purpose of the connection. For example, the arms 14 may be arranged other than at the periphery of the cylindrical body 11. The arms 14 may extend into the male part 12 and the groove 30 could be provided on the inner surface of the male part 12. Other arrangements of the mechanical elements may be used to create a coupling that detaches when subjected to a predetermined separating force, for example through a magnetic coupling, a friction fit, or a semi-perforated tape or other adhesive.

Although the above embodiments have been described in association with medical fluid lines, the present invention is not limited to fluid lines and may be used in connection with other medical lines, such as oxygen lines.

Other modifications or adaptations will be apparent to those of ordinary skill in the art.

The present invention may be embodied in other specific forms without departing from the claimed characteristics thereof. Therefore, the above discussed embodiments are considered to be illustrative and not restrictive, the scope of the invention being indicated by the appended claims rather than the foregoing description.

## Claims

1. A coupling device (8, 108, 208, 308, 408, 508) for coupling a patient-side medical line to an equipment-side medical line, said coupling device comprising:
a first part (10) adapted to be coupled to a first medical line (22), said first part (10) having a first passage (20) therethrough to provide fluid communication with said first medical line (22), said first part including a first seal (18) having a sealed position and an unsealed position, wherein said first seal (18) seals said first medical line (22) when in said sealed position; and
a second part (12) adapted to be coupled to a second medical line (23), said second part (12) having a second passage (26) therethrough to provide fluid communication with said second medical line (23), said second part (12) including a second seal (28) having a sealed position and an unsealed position, wherein said second seal (28) seals said second medical line (23) when in said sealed position, said second seal including an integral sheath (19) and forwardly extending skirt (21) for enveloping a fluid delivery device (24) coupled to said second medical line (23) and received in said second passage (26), wherein said sheath (19) and said skirt (21) are formed of sufficiently resiliently elastic materials; and
wherein said parts (10, 12) include a connector (14, 29) detachably connecting said first part (10) to said second part (12) in a longitudinal direction, said first part (10) receiving said second part (12), wherein said connector detaches said first and second parts (10, 12) in response to a predetermined force in said longitudinal direction, and wherein each of said seals (18, 28) moves from said sealed position to said unsealed position when said first part (10) is detachably connected to said second part (12), said first part (10) including a protrusion (15) for pushing said second sealing means (28) into an unsealed position to establish fluid communication between said first and second parts (10, 12) in a single action when said first and second parts (10, 12) are connected.

2. The coupling device (8, 108, 208, 308, 408, 508) claimed in claim 1, wherein said connector (14, 29) includes a snap-fit connector (16, 30).

3. The coupling device (8, 108, 208, 308, 408, 508) claimed in claim 1, wherein said connector includes a rounded protrusion (16) on said first part (10) and a cooperating groove (30) formed in the surface of said second part (12).

4. The coupling device (8, 108, 208, 308, 408, 508) claimed in claim 3, wherein said first part (10) includes one or more resiliently flexible arms (14) extending longitudinally and having formed thereon one or more of said protrusions (16).

5. The coupling device (8, 108, 208, 308, 408, 508) claimed in claim 4, wherein said first part (10) includes a cylindrical body (11), said arms (14) are disposed around the periphery of said cylindrical body (11), and said protrusions (16) extend radially inwards, and wherein said second part (12) includes a cylindrical main body (13) and said cooperating groove (30) extends circumferentially within the outer surface of said cylindrical main body (13).

6. The coupling device (8, 108, 208, 308, 408, 508) claimed in claim 1, wherein at least one of said seals (18, 28) includes a diaphragm.

7. The coupling device (8, 108, 208, 308, 408, 508) claimed in claim 6, wherein said diaphragm (18, 28) is formed from silicone.

8. The coupling device (8, 108, 208, 308, 408, 508) claimed in claim 6, wherein said diaphragm (18, 28) includes a slit (54) therethrough, and wherein in said sealed position said diaphragm (18, 28) pinches said slit (54) closed, and in said unsealed position said diaphragm (18, 28) draws apart at said slit (54).

9. The coupling device (8, 108, 208, 308, 408, 508) claimed in claim 8, wherein said diaphragm (18, 28) has an inner surface (50) and includes a pair of channels (56) formed within said surface on either side of said slit (54).

10. The coupling device (8, 108, 208, 308, 408, 508) claimed in claim 9, wherein said channels (56) are parallel to said slit (54), and wherein said channels (56) each include an angled face (58) having a surface angled with respect to said slit (54).

11. The coupling device (8, 108, 208, 308, 408, 508) claimed in claim 8, wherein said diaphragm has an inner surface (50) and includes a circumferential channel (56) formed within said surface around said slit (54).

12. The coupling device (8, 108, 208, 308, 408, 508) claimed in claim 11, wherein said circumferential channel (56) includes an angled face (58) having a surface angled with respect to said slit (54).

13. The coupling device (8, 108, 208, 308, 408, 508) claimed in claim 1, wherein said fluid delivery device (24) includes a needle.

14. The coupling device (8, 108, 208, 308, 408, 508) claimed in claim 1, wherein the end of said sheath (19) includes said diaphragm (28) having said slit (54) therein.

15. The coupling device (8, 108, 208, 308, 408, 508) claimed in claim 1, wherein said skirt (21) has an outer end (27) coupled to the second part (12).

16. The coupling device (8, 108, 208, 308, 408, 508) claimed in claim 1, wherein said fluid delivery device (24) of said second part (12) pushes said first sealing means 18 into an unsealed position when said first and second parts (10, 12) are connected.

17. A coupling device (108) as claimed in claim 1, wherein:
said first and second parts (10, 12) are rotatable with respect to each other when said first and second parts (10, 12) are connected,
said first and second parts (10, 12) being rotatable to a plurality of locking positions, each of said locking positions corresponding to a different, predetermined force for detaching said first part (10) from said second part (12) in response thereto.

18. A coupling device (108) as claimed in claim 17, wherein each of said locking positions corresponds to an increasing, predetermined force.

19. A coupling device (108) as claimed in claims 17 or 18, wherein said coupling device (108) includes alignment markings (70, 78, 80, 82) corresponding to each of said plurality of locking positions.

20. A coupling device (108) as claimed in claim 17, wherein:
the first part (10) includes a cylindrical body (11) having a plurality of resiliently flexible arms (14) disposed around the periphery of said cylindrical body (11) and extending longitudinally therefrom,
a first set of said arms having a first, radially inwardly projecting ridge (16) formed on the inner surface thereof, and
a second set of said arms (14) having said first ridge (16) and a second, inwardly projecting ridge (116) formed on the inner surface thereof,
the second ridge (116) being rearwardly disposed with respect to said first ridge (16) on the inner surface of said arms (14); and
the second part (12) includes a cylindrical body (13) having inwardly tapered engagement surfaces (74) and inwardly sloped clearance surfaces (76) at the proximal end thereof, and a circumferential groove (30) formed on the outer surface of said cylindrical body (13), each of said inwardly tapered engagement surfaces (74) having a secondary circumferential groove (72) formed thereon, said secondary circumferential groove (72) being forwardly disposed with respect to said circumferential groove (30);
wherein said first and second parts (10, 12) are detachably connected in a first locking position wherein said first set of arms (14) are aligned with said clearance surfaces (76),
said first ridge (16) engaging said circumferential groove (30), said first locking position requiring a corresponding first predetermined force for detaching said first and second parts (10, 12); and
whereby rotation of one of said first and second parts (10, 12) brings said second set of arms into contact with said inwardly tapered engagement surfaces (74) thereby engaging at least a portion of each of said second ridges (116) with the corresponding secondary circumferential groove (72), the engagement between said second ridges (116) and said secondary grooves (72) corresponding to an increased predetermined force for detaching said first and second parts (10, 12).

21. A coupling device (108) as claimed in claim 20, wherein said coupling device (108) includes alignment markings (70, 78, 80, 82) corresponding to each of said plurality of locking positions.

22. A coupling device (108) as claimed in claim 20, wherein said alignment markings (70, 78, 80, 82) comprise an alignment arrow (70) formed on the outside surface of said first part (10), and a series of alignment identifiers (78, 80, 82) formed on the outside surface of said second part (12), said alignment arrow (70) aligning with one of said alignment identifiers (78, 80, 82) when said first part (10) is connected to said second part (12) in one of said locking positions, the series of alignment identifiers (78, 80, 82) thereby distinguishing the different, predetermined force associated with each of said locking positions.

23. The coupling device (108) as claimed in claim 22, wherein each of said series of alignment identifiers (78, 80, 82) corresponds to a location of said second ridges (116) with respect to said secondary grooves (72) when said first and second parts (10, 12) are coupled together and said alignment arrow (70) aligns with a particular one of said alignment identifiers (78, 80, 82).

24. The coupling device (108) as claimed in any one of claims 20 to 23, further including a stop mechanism for preventing further rotation of one of said first and second parts (10, 12) with respect to the other of said parts (10, 12).

25. The coupling device (108) as claimed in any one of claims 22 to 24, further including a click mechanism for providing audible confirmation that said alignment arrow (70) on said first part (10) is appropriately aligned with a corresponding alignment identifier (78, 80, 82) on said second part (12) when said first and second parts (10, 12) are coupled together.

26. The coupling device (8, 108, 208, 308, 408, 508) as claimed in any one of claims 1 to 21, wherein said second part (12) includes an integrated backflow prevention mechanism (90) to ensure fluid flows in only one direction through said coupling device.

27. The coupling device (208) as claimed in claim 26, wherein said backflow prevention mechanism (90) includes a filter element (100) for preventing the flow of infectious disease agents through said coupling device.

28. The coupling device (208) as claimed in claim 22, wherein said backflow prevention mechanism (90) is selected from the group comprising: a disk valve body (96), a ball-type valve body (110), and a pinch-type valve body (120).

29. The coupling device (8, 108, 208, 308, 408, 508) as claimed in any one of claims 1 to 28, further including an external med-port body (136), said external med-port body (136) having a first fluid passage (138) in fluid communication with said first and second passages (20, 26) when said first and second parts (10, 12) are detachably connected together, said external med-port body (136) further including a second fluid passage (134) in fluid communication with said first fluid passage (138), said second fluid passage (134) being oriented tangentially with respect to said first fluid passage (138) and permitting a secondary fluid to be introduced into said first and second medical lines (22, 23).

## Patentansprüche

1. Kopplungsvorrichtung (8, 108, 208, 308, 408, 508) um eine medizinische Leitung am Patientenort mit einer medizinischen Leitung am Geräteort zu verbinden, wobei die Kopplungsvorrichtung umfasst:
ein erstes Teilstück (10), welches eingerichtet ist, um mit einer ersten medizinischen Leitung (22) verbunden zu werden, wobei das erste Teilstück einen ersten Durchgang (20) hat, um hierdurch Austausch von Flüssigkeit mit der zweiten medizinischen Leitung (22) bereitzustellen, wobei das erste Teilstück eine erste Dichtung (18) beinhaltet, die eine verschlossene Position und eine unverschlossene Position hat, wobei die erste Dichtung (18) die erste medizinische Leitung (22) verschließt, wenn sie sich in der verschlossenen Position befindet; und
ein zweites Teilstück (12), welches eingerichtet ist, um mit einer zweiten medizinischen Leitung (23) verbunden zu werden, wobei das zweite Teilstück (12) einen zweiten Durchgang (26) hat, um hierdurch Austausch von Flüssigkeit mit der zweiten medizinischen Leitung (23) bereitzustellen, wobei das zweite Teilstück (12) eine zweite Dichtung (28) beinhaltet, die eine verschlossene Position und eine unverschlossene Position hat, wobei die zweite Dichtung (28) die zweite medizinische Leitung (23) verschließt, wenn sie sich in der verschlossenen Position befindet, wobei die zweite Dichtung eine integrale Umhüllung (19) und eine nach vorne ausladende Schürze (21) beinhaltet, um eine Vorrichtung zur Flüssigkeits-Übergabe (24) zu umhüllen, die mit der zweiten medizinischen Leitung (23) verbunden ist und in dem zweiten Durchgang (26) erhalten ist, wobei die Umhüllung (19) und die Schürze (21) aus ausreichend robusten, elastischen Materialien geformt sind; und
wobei die Teilstücke (10, 12) einen Steckverbinder (14, 29) beinhalten, um das erste Teilstück (10) mit dem zweiten Teilstück (12) lösbar in longitudinaler Richtung zu verbinden, wobei das erste Teilstück (10) das zweite Teilstück (12) aufnimmt, wobei der Steckverbinder das erste und zweite Teilstück (10, 12) als Reaktion auf eine vorbestimmte Kraft in die longitudinale Richtung voneinander löst, und wobei jede der Dichtungen (18, 28) sich von der verschlossenen Position zu der unverschlossenen Position bewegt, wenn das erste Teilstück (10) lösbar mit dem zweiten Teilstück (12) verbunden wird, wobei das erste Teilstück (10) eine Ausbuchtung (15) beinhaltet, um das zweite Mittel zur Dichtung (28) in eine unverschlossene Position zu drücken, um einen Austausch von Flüssigkeit zwischen dem ersten und zweiten Teilstück (10, 12) in einem einzigen Arbeitsgang aufzubauen, wenn das erste und zweite Teilstück (10, 12) verbunden werden.

2. Die Kopplungsvorrichtung (8, 108, 208, 308, 408, 508) nach Anspruch 1, wobei der Steckverbinder (14, 29) einen Einschnapp-Steckverbinder (16, 30) beinhaltet.

3. Die Kopplungsvorrichtung (8, 108, 208, 308, 408, 508) nach Anspruch 1, wobei der Steckverbinder eine abgerundete Ausbuchtung (16) an dem ersten Teilstück (10) und eine Zusammenwirkungs-Nut (30), welche in der Oberfläche des zweiten Teilstücks (12) ausgebildet ist, beinhaltet.

4. Die Kopplungsvorrichtung (8, 108, 208, 308, 408, 508) nach Anspruch 3, wobei das erste Teilstück (10) einen oder mehrere robuste biegbare Arme (14) beinhaltet, die sich longitudinal erstrecken und die eine oder mehrere der Ausbuchtungen (16) darauf ausgebildet haben.

5. Die Kopplungsvorrichtung (8, 108, 208, 308, 408, 508) nach Anspruch 4, wobei das erste Teilstück (10) einen zylindrischen Körper (11) beinhaltet, wobei die Arme (14) um den Umfang des zylindrischen Körpers (11) angeordnet sind, und die Ausbuchtungen (16) sternförmig nach innen ragen und wobei das zweite Teilstück (12) einen zylindrischen Hauptkörper (13) beinhaltet und die Zusammenwirkungs-Nut (30) sich umlaufend innerhalb der äußeren Oberfläche des zylindrischen Hauptkörpers (13) erstreckt.

6. Die Kopplungsvorrichtung (8, 108, 208, 308, 408, 508) nach Anspruch 1, wobei mindestens eine der Dichtungen (18, 28) eine Membran beinhaltet.

7. Die Kopplungsvorrichtung (8, 108, 208, 308, 408, 508) nach Anspruch 6, wobei die Membran (18, 28) aus Silikon ausgebildet ist.

8. Die Kopplungsvorrichtung (8, 108, 208, 308, 408, 508) nach Anspruch 6, wobei die Membran (18, 28) einen Spalt (54) hindurch beinhaltet, und wobei in der geschlossenen Position die Membran (18, 28) den Spalt (54) zudrückt, und in der unverschlossenen Position die Membran (18, 28) an dem Spalt (54) auseinandergezogen wird.

9. Die Kopplungsvorrichtung (8, 108, 208, 308, 408, 508) nach Anspruch 8, wobei die Membran (18, 28) eine innere Oberfläche (50) hat und ein Paar Kanäle (56), die innerhalb der Oberfläche auf beiden Seiten des Spaltes (54) ausgebildet sind.

10. Die Kopplungsvorrichtung (8, 108, 208, 308, 408, 508) nach Anspruch 9, wobei die Kanäle (56) parallel zu dem Spalt (54) sind und wobei die Kanäle (56) jeweils eine angewinkelte Vorderseite (58) beinhalten, die eine im Verhältnis zu dem Spalt (54) gewinkelte Oberfläche haben.

11. Die Kopplungsvorrichtung (8, 108, 208, 308, 408, 508) nach Anspruch 8, wobei die Membran eine innere Oberfläche (50) hat und einen umlaufenden Kanal (56) beinhaltet, der innerhalb der Oberfläche um den Spalt (54)ausgebildet ist.

12. Die Kopplungsvorrichtung (8, 108, 208, 308, 408, 508) nach Anspruch 11, wobei der umlaufende Kanal (56) eine angewinkelte Vorderseite (58) beinhaltet, die eine im Verhältnis zu dem Spalt (54) angewinkelte Oberfläche hat.

13. Die Kopplungsvorrichtung (8, 108, 208, 308, 408, 508) nach Anspruch 1, wobei die Vorrichtung zur Flüssigkeits-Übergabe (24) eine Nadel umfasst.

14. Die Kopplungsvorrichtung (8, 108, 208, 308, 408, 508) nach Anspruch 1, wobei das Ende der Umhüllung (19) die Membran (28) beinhaltet, welche den Spalt (54) darin hat.

15. Die Kopplungsvorrichtung (8, 108, 208, 308, 408, 508) nach Anspruch 1, wobei die Schürze (21) ein äußeres Ende (27) hat, das mit dem zweiten Teilstück (12) verbunden ist.

16. Die Kopplungsvorrichtung (8, 108, 208, 308, 408, 508) nach Anspruch 1, wobei die Vorrichtung zur Flüssigkeits-Übergabe (24) von dem zweiten Teilstück (12) das erste Dichtungsmittel (18) in eine unverschlossene Position drückt, wenn das erste und zweite Teilstück (10, 12) verbunden werden.

17. Kopplungsvorrichtung (108) nach Anspruch 1, wobei:
das erste und zweite Teilstück (10, 12) gegeneinander verdrehbar sind, wenn das erste und zweite Teilstück (10, 12) verbunden sind,
das erste und zweite Teilstück (10, 12) verdrehbar auf eine Vielzahl von Verriegelungspositionen ist, wobei jede dieser Verriegelungspositionen einer anderen, vorbestimmten Kraft entspricht, um das erste Teilstück (10) von dem zweiten Teilstück (12) als Reaktion darauf zu lösen.

18. Kopplungsvorrichtung (108) nach Anspruch 17, wobei jede dieser Verriegelungspositionen einer ansteigenden, vorbestimmten Kraft entspricht.

19. Kopplungsvorrichtung (108) nach Anspruch 17 oder 18, wobei die Kopplungsvorrichtung (108) Ausrichtungsmarkierungen (70, 78, 80, 82) beinhaltet, die den jeweiligen der Vielzahl von Verriegelungspositionen entsprechen.

20. Kopplungsvorrichtung (108) nach Anspruch 17, wobei:
das erstes Teilstück (10) einen zylindrischen Körper (11) beinhaltet, welcher eine Vielzahl von robusten, flexiblen Armen (14) hat, welche um den Umfang des zylindrischen Körpers (11) angeordnet sind und sich longitudinal von ihm erstrecken,
ein erster Satz von Armen, welcher einen ersten, radial nach innen vorstehenden Grat (16) hat, der auf der inneren Oberfläche hiervon ausgebildet ist; und
ein zweiter Satz von Armen (14), welcher den ersten Grat (16) hat und einen zweiten, nach innen vorstehenden Grat (116), der auf der inneren Oberfläche hiervon ausgebildet ist,
der zweite Grat (116), welcher nach hinten im Verhältnis zu dem ersten Grat (16) auf der inneren Oberfläche der Arme (14) versetzt ist; und
das zweite Teilstück (12) einen zylindrischen Körper (13) beinhaltet, der nach innen kegelförmige Kupplungs-Oberflächen (74) hat und nach innen abgeschrägte Freigabe-Oberflächen (76) an seiner proximalen Seite hat, und eine umlaufende Nut (30), die auf der äußeren Oberfläche des zylindrischen Körpers (13) ausgebildet ist, wobei jede der nach innen kegelförmigen Kupplungs-Oberflächen (74) eine zweite umlaufende Nut (72) hat, die darauf ausgeformt ist, wobei die zweite umlaufende Nut (72) nach vorne versetzt ist im Verhältnis zu der umlaufenden Nut (30);
wobei das erste und zweite Teilstück (10, 12) lösbar verbunden sind in einer erste Verriegelungsposition, wobei der erste Satz von Armen (14) zu den Freigabe-Oberflächen (76) ausgerichtet ist;
der erste Grat (16) welcher in die umlaufende Nut (30) einrastet, wobei die erste Verriegelungsposition eine entsprechende erste vorbestimmte Kraft zum Lösen des ersten vom zweiten Teilstück (10, 12) erfordert; und
wobei eine Drehung von einem, dem ersten oder zweiten Teilstück (10, 12) den zweiten Satz von Armen in Kontakt mit den nach innen kegelförmigen Kupplungs-Oberflächen (74) bringt und **dadurch** mindestens einen Teil von jedem der zweiten Grate (116) mit der entsprechenden zweiten umlaufenden Nut (72) verrastet, wobei die Befestigung zwischen den zweiten Graten (116) und den zweiten Nuten (72) einer erhöhten vorbestimmten Kraft zum Lösen des ersten vom zweiten Teilstück (10, 12) entspricht.

21. Kopplungsvorrichtung (108) nach Anspruch 20, wobei die Kopplungsvorrichtung (108) Ausrichtungsmarkierungen (70, 78, 80, 82) entsprechend jeder der Vielzahl von Verriegelungspositionen beinhaltet.

22. Kopplungsvorrichtung (108) nach Anspruch 20, wobei die Ausrichtungsmarkierungen (70, 78, 80, 82) einen Ausrichtungspfeil (70) umfassen, der auf der äußeren Oberfläche des ersten Teilstücks (10) ausgebildet ist, und eine Reihe von Ausrichtungskennungen (78, 80, 82) welche auf der äußeren Oberfläche des zweiten Teilstücks (12) ausgebildet sind, wobei der Ausrichtungspfeil (70) ausgerichtet ist mit einer der Ausrichtungskennungen (78, 80, 82), wenn das erste Teilstück (10) mit dem zweiten Teilstück (12) in einer der Verriegelungspositionen verbunden ist, wobei die Reihe von Ausrichtungskennungen (78, 80, 82) dabei die verschiedene, vorbestimmte Kraft unterscheidet, welche mit jeder der Verriegelungspositionen verknüpft ist.

23. Die Kopplungsvorrichtung (108) nach Anspruch 22, wobei jede der Reihe von Ausrichtungskennungen (78, 80, 82) einen Ort der zweiten Grate (116) im Verhältnis zu den zweiten Nuten (72) entspricht, wenn das erste und zweite Teilstück (10, 12) miteinander verbunden sind und der Ausrichtungspfeil (70) mit einer einzelnen der Ausrichtungskennungen (78, 80, 82) ausgerichtet ist.

24. Die Kopplungsvorrichtung (108) nach einem der Ansprüche 20 bis 23, weiterhin einen Stoppmechanismus beinhaltend, um eine weitere Drehung von einem der ersten oder zweiten Teilstücke (10, 12) im Verhältnis zu dem anderen der Teilstücke zu verhindern.

25. Die Kopplungsvorrichtung (108) nach einem der Ansprüche 22 bis 24, weiterhin einen Klickmechanismus beinhaltend, um eine hörbare Bestätigung bereitzustellen, dass der Ausrichtungspfeil (70) auf dem ersten Teilstück (10) richtig ausgerichtet ist zu einer entsprechenden Ausrichtungskennung (78, 80, 82) auf dem zweiten Teilstück (12), wenn das erste und zweite Teilstück (10, 12) miteinander verbunden sind.

26. Die Kopplungsvorrichtung (8, 108, 208, 308, 408, 508) nach einem der Ansprüche 1 bis 21, wobei das zweite Teilstück (12) einen integrierten Rückflussverhinderungsmechanismus (90) beinhaltet, um sicherzustellen, dass Flüssigkeit nur in eine Richtung durch die Kopplungsvorrichtung fließt.

27. Die Kopplungsvorrichtung (208) nach Anspruch 26, wobei der Rückflussverhinderungsmechanismus (90) ein Filterelement (100) beinhaltet, um den Durchfluss von infektiösen Krankheitserregern durch die Kopplungsvorrichtung zu verhindern.

28. Die Kopplungsvorrichtung (208) nach Anspruch 22, wobei der Rückflussverhinderungsmechanismus (90) ausgewählt ist von einer Gruppe bestehend aus: ein Scheibenventilkörper (96), ein Kugelventilkörper (110) und ein Quetschventilkörper (120).

29. Die Kopplungsvorrichtung (8, 108, 208, 308, 408, 508) nach einem der Ansprüche 1 bis 28, weiterhin einen externen Med-Port-Körper (136) beinhaltend, wobei der externe Med-Port-Körper (136) einen ersten Flüssigkeitsdurchgang (138) mit einem Austausch von Flüssigkeit mit dem ersten und zweiten Durchgang (20, 26) hat, wenn das erste und zweite Teilstück (10, 12) lösbar miteinander verbunden sind, wobei der externe Med-Port-Körper (136) weiterhin eine zweiten Flüssigkeitsdurchgang (134) mit einem Austausch von Flüssigkeit mit dem ersten Flüssigkeitsdurchgang (138) beinhaltet, wobei der zweite Flüssigkeitsdurchgang (134) tangential im Verhältnis zum ersten Flüssigkeitsdurchgang (138) ausgerichtet ist und einer zweiten Flüssigkeit gestattet in die erste und zweite medizinische Leitung (22, 23) geleitet zu werden.

## Revendications

1. Dispositif de couplage (8, 108, 208, 308, 408, 508) pour coupler un tuyau médical du côté du patient à un tuyau médical du côté de l'équipement, ledit dispositif de couplage comprenant :
une première partie (10) adaptée pour être couplée à un premier tuyau médical (22), ladite première partie (10) ayant un premier passage (20) à travers ce dernier afin de fournir la communication de fluide avec ledit premier tuyau médical (22), ladite première partie comprenant un premier joint d'étanchéité (18) ayant une position étanche et une position non étanche, dans lequel ledit premier joint d'étanchéité (18) réalise l'étanchéité dudit premier tuyau médical (22) lorsqu'il est dans ladite position étanche ; et
une deuxième partie (12) adaptée pour être couplée à un deuxième tuyau médical (23), ladite deuxième partie (12) ayant un deuxième passage (26) à travers ce dernier afin de fournir la communication de fluide avec ledit deuxième tuyau médical (23), ladite deuxième partie (12) comprenant un deuxième joint d'étanchéité (28) ayant une position étanche et une position non étanche, dans lequel ledit deuxième joint d'étanchéité (28) réalise l'étanchéité dudit deuxième tuyau médical (23) lorsqu'il est dans ladite position étanche, ledit deuxième joint d'étanchéité comprenant une gaine solidaire (19) et une jupe s'étendant vers l'avant (21) pour envelopper un dispositif de distribution de fluide (24) couplé audit deuxième tuyau médical (23) et reçu dans ledit deuxième passage (26), dans lequel ladite gaine (19) et ladite jupe (21) sont formées avec des matériaux élastiques suffisamment résilients ; et
dans lequel lesdites parties (10, 12) comprennent un connecteur (14, 29) raccordant de manière détachable ladite première partie (10) à ladite deuxième partie (12) dans une direction longitudinale, ladite première partie (10) recevant ladite deuxième partie (12), dans lequel ledit connecteur détache lesdites première et deuxième parties (10, 12) en réponse à une force prédéterminée dans ladite direction longitudinale, et dans lequel chacun desdits joints d'étanchéité (18, 28) passe de ladite position étanche à ladite position non étanche lorsque ladite première partie (10) est raccordée de manière détachable à ladite deuxième partie (12), ladite première partie (10) comprenant une saillie (15) pour pousser lesdits deuxièmes moyens d'étanchéité (28) dans la position non étanche afin d'établir une communication de fluide entre lesdites première et deuxième parties (10, 12) en une seule action, lorsque lesdites première et deuxième parties (10, 12) sont raccordées.

2. Dispositif de couplage (8, 108, 208, 308, 408, 508) selon la revendication 1, dans lequel ledit connecteur (14, 29) comprend un connecteur à encliquetage (16, 30).

3. Dispositif de couplage (8, 108, 208, 308, 408, 508) selon la revendication 1, dans lequel ledit connecteur comprend une saillie arrondie (16) sur ladite première partie (10) et une rainure de coopération (30) formée dans la surface de ladite deuxième partie (12).

4. Dispositif de couplage (8, 108, 208, 308, 408, 508) selon la revendication 3, dans lequel ladite première partie (10) comprend un ou plusieurs bras élastiquement flexibles (14) s'étendant longitudinalement et ayant formé sur ces derniers, une ou plusieurs desdites saillies (16).

5. Dispositif de couplage (8, 108, 208, 308, 408, 508) selon la revendication 4, dans lequel ladite première partie (10) comprend un corps cylindrique (11), lesdits bras (14) sont disposés autour de la périphérie dudit corps cylindrique (11), et lesdites saillies (16) s'étendent radialement vers l'intérieur, et dans lequel ladite deuxième partie (12) comprend un corps principal cylindrique (13) et ladite rainure de coopération (30) s'étend de manière circonférentielle à l'intérieur de la surface externe dudit corps principal cylindrique (13).

6. Dispositif de couplage (8, 108, 208, 308, 408, 508) selon la revendication 1, dans lequel au moins l'un desdits joints d'étanchéité (18, 28) comprend un diaphragme.

7. Dispositif de couplage (8, 108, 208, 308, 408, 508) selon la revendication 6, dans lequel ledit diaphragme (18, 28) est formé à partir de silicone.

8. Dispositif de couplage (8, 108, 208, 308, 408, 508) selon la revendication 6, dans lequel ledit diaphragme (18, 28) comprend une fente (54) à travers ce dernier, et dans lequel, dans ladite position étanche, ledit diaphragme (18, 28) pince ladite fente (54) pour la fermer, et dans ladite position non étanche, ledit diaphragme (18, 28) s'écarte au niveau de ladite fente (54).

9. Dispositif de couplage (8, 108, 208, 308, 408, 508) selon la revendication 8, dans lequel ledit diaphragme (18, 28) a une surface interne (50) et comprend une paire de canaux (56) formés à l'intérieur de ladite surface de chaque côté de ladite fente (54).

10. Dispositif de couplage (8, 108, 208, 308, 408, 508) selon la revendication 9, dans lequel lesdits canaux (56) sont parallèles à ladite fente (54) et dans lequel lesdits canaux (56) comprennent chacun une face coudée (58) ayant une surface coudée par rapport à ladite fente (54).

11. Dispositif de couplage (8, 108, 208, 308, 408, 508) selon la revendication 8, dans lequel ledit diaphragme a une surface interne (50) et comprend un canal circonférentiel (56) formé à l'intérieur de ladite surface autour de ladite fente (54).

12. Dispositif de couplage (8, 108, 208, 308, 408, 508) selon la revendication 11, dans lequel ledit canal circonférentiel (56) comprend une face coudée (58) ayant une surface coudée par rapport à ladite fente (54).

13. Dispositif de couplage (8, 108, 208, 308, 408, 508) selon la revendication 1, dans lequel ledit dispositif de distribution de fluide (24) comprend une aiguille.

14. Dispositif de couplage (8, 108, 208, 308, 408, 508) selon la revendication 1, dans lequel l'extrémité de ladite gaine (19) comprend ledit diaphragme (28) ayant ladite fente (54) à l'intérieur de cette dernière.

15. Dispositif de couplage (8, 108, 208, 308, 408, 508) selon la revendication 1, dans lequel ladite jupe (21) a une extrémité externe (27) couplée à la deuxième partie (12).

16. Dispositif de couplage (8, 108, 208, 308, 408, 508) selon la revendication 1, dans lequel ledit dispositif de distribution de fluide (24) de ladite deuxième partie (12) pousse lesdits premiers moyens d'étanchéité (18) dans une position non étanche lorsque lesdites première et deuxième parties (10, 12) sont raccordées.

17. Dispositif de couplage (108) selon la revendication 1, dans lequel :
lesdites première et deuxième parties (10, 12) peuvent tourner l'une par rapport à l'autre lorsque lesdites première et deuxième parties (10, 12) sont raccordées,
lesdites première et deuxième parties (10, 12) étant entraînées en rotation jusqu'à une pluralité de positions de blocage, chacune desdites positions de blocage correspondant à une force prédéterminée différente pour détacher ladite première partie (10) de ladite deuxième partie (12) en réponse à cela.

18. Dispositif de couplage (108) selon la revendication 17, dans lequel chacune desdites positions de blocage correspond à une force prédéterminée croissante.

19. Dispositif de couplage (108) selon les revendications 17 ou 18, dans lequel ledit dispositif de couplage (108) comprend des marques d'alignement (70, 78, 80, 82) correspondant à chacune de ladite pluralité de positions de blocage.

20. Dispositif de couplage (108) selon la revendication 17, dans lequel :
la première partie (10) comprend un corps cylindrique (11) ayant une pluralité de bras élastiquement flexibles (14) disposés autour de la périphérie dudit corps cylindrique (11) et s'étendant latéralement à partir de ce dernier,
un premier ensemble desdits bras ayant une première crête en saillie radialement vers l'intérieur (16) formée sur sa surface interne, et
un deuxième ensemble desdits bras (14) ayant ladite première crête (16) et une deuxième crête en saillie vers l'intérieur (116) formée sur sa surface interne,
la deuxième crête (116) étant disposée vers l'arrière par rapport à ladite première crête (16) sur la surface interne desdits bras (14) ; et
la deuxième partie (12) comprend un corps cylindrique (13) ayant des surfaces de mise en prise progressivement rétrécies vers l'intérieur (74) et des surfaces de jeu inclinées vers l'intérieur (76) au niveau de son extrémité proximale, et une rainure circonférentielle (30) formée sur la surface externe dudit corps cylindrique (13), chacune desdites surfaces de mise en prise progressivement rétrécies vers l'intérieur (74) ayant une rainure circonférentielle secondaire (72) formée sur cette dernière, ladite rainure circonférentielle secondaire (72) étant disposée vers l'avant par rapport à ladite rainure circonférentielle (30) ;
dans lequel lesdites première et deuxième parties (10, 12) sont raccordées de manière détachable dans une première position de blocage, dans laquelle ledit premier ensemble de bras (14) est aligné avec lesdites surfaces de jeu (76),
ladite première crête (16) mettant en prise ladite rainure circonférentielle (30), ladite première position de blocage nécessitant une première force prédéterminée correspondante pour détacher lesdites première et deuxième parties (10, 12) ; et
moyennant quoi la rotation de l'une desdites première et deuxième parties (10, 12) amène ledit deuxième ensemble de bras en contact avec lesdites surfaces de mise en prise progressivement rétrécies vers l'intérieur (74) mettant ainsi en prise au moins une partie de chacune desdites deuxièmes crêtes (116) avec la rainure circonférentielle secondaire (72) correspondante, la mise en prise entre lesdites deuxièmes crêtes (116) et lesdites deuxièmes rainures (72) correspondant à une force prédéterminée accrue pour détacher lesdites première et deuxième parties (10, 12).

21. Dispositif de couplage (108) selon la revendication 20, dans lequel ledit dispositif de couplage (108) comprend des marques d'alignement (70, 78, 80, 82) correspondant à chacune de ladite pluralité de positions de blocage.

22. Dispositif de couplage (108) selon la revendication 20, dans lequel lesdites marques d'alignement (70, 78, 80, 82) comprennent une flèche d'alignement (70) formée sur la surface externe de ladite première partie (10) et une série de dispositifs d'identification d'alignement (78, 80, 82) formés sur la surface externe de ladite deuxième partie (12), ladite flèche d'alignement (70) s'alignant avec l'un desdits dispositifs d'identification d'alignement (78, 80, 82) lorsque ladite première partie (10) est raccordée à ladite deuxième partie (12) dans l'une desdites positions de blocage, la série de dispositifs d'identification d'alignement (78, 80, 82) distinguant ainsi la force prédéterminée différente associée à chacune desdites positions de blocage.

23. Dispositif de couplage (108) selon la revendication 22, dans lequel chacun de ladite série de dispositifs d'identification d'alignement (78, 80, 82) correspond à un emplacement desdites deuxièmes crêtes (116) par rapport auxdites rainures secondaires (72) lorsque lesdites première et deuxième parties (10, 12) sont couplées ensemble et ladite flèche d'alignement (70) s'aligne avec un dispositif particulier desdits dispositifs d'identification d'alignement (78, 80, 82).

24. Dispositif de couplage (108) selon l'une quelconque des revendications 20 à 23, comprenant en outre un mécanisme de butée pour empêcher la rotation supplémentaire de l'une desdites première et deuxième parties (10, 12) par rapport à l'autre desdites parties (10, 12).

25. Dispositif de couplage (108) selon l'une quelconque des revendications 22 à 24, comprenant en outre un mécanisme d'encliquetage pour fournir une information audible que ladite flèche d'alignement (70) sur ladite première partie (10) est alignée de manière appropriée avec un dispositif d'identification d'alignement (78, 80, 82) correspondant sur ladite deuxième partie (12) lorsque lesdites première et deuxième parties (10, 12) sont couplées ensemble.

26. Dispositif de couplage (8, 108, 208, 308, 408, 508) selon l'une quelconque des revendications 1 à 21, dans lequel ladite deuxième partie (12) comprend un mécanisme anti-refoulement intégré (90) pour garantir que le fluide s'écoule dans une seule direction à travers ledit dispositif de couplage.

27. Dispositif de couplage (208) selon la revendication 26, dans lequel ledit mécanisme anti-refoulement (90) comprend un élément de filtre (100) pour empêcher l'écoulement d'agents pathologiques infectieux à travers ledit dispositif de couplage.

28. Dispositif de couplage (208) selon la revendication 22, dans lequel ledit mécanisme anti-refoulement (90) est choisi dans le groupe comprenant : un corps de soupape à disque (96), un corps de soupape de type à clapet (110) et un corps de soupape de type à pincement (120).

29. Dispositif de couplage (8, 108, 208, 308, 408, 508) selon l'une quelconque des revendications 1 à 28, comprenant en outre un corps d'orifice médical externe (136), ledit corps d'orifice médical externe (136) ayant un premier passage (138) en communication de fluide avec lesdits premier et deuxième passages (20, 26) lorsque lesdites première et deuxième parties (10, 12) sont raccordées de manière détachable ensemble, ledit corps d'orifice médical externe (136) comprenant en outre un deuxième passage de fluide (134) en communication de fluide avec ledit premier passage de fluide (138), ledit deuxième passage de fluide (134) étant orienté de manière tangentielle par rapport audit premier passage de fluide (138) et permettant d'introduire un fluide secondaire dans lesdits premier et deuxième tuyaux médicaux (22, 23).
